# EUROPEAN PATENT SPECIFICATION

(11) **EP 2 718 453 B1**
(45) Date of publication and mention of the grant of the patent: **03.12.2014**
(21) Application number: 12737610.1
(22) Date of filing: 28.05.2012
(51) Int. Cl.: C12P 7/64, C10L 1/02

(54) **ENGINE WORTHY FATTY ACID METHYL ESTER (BIODIESEL) FROM NATURALLY OCCURRING MARINE MICROALGAL MATS AND MARINE MICROALGAE CULTURED IN OPEN SALT PANS TOGETHER WITH VALUE ADDITION OF CO-PRODUCTS**
MOTORGERECHTER FETTSÄURE-METHYLESTER (BIODIESEL) AUS NATÜRLICH VORKOMMENDEN UND IN OFFENEN SALZPFANNEN UNTER BEIGABE VON NEBENPRODUKTEN GEZÜCHTETEN MEERESMIKROALGENMATTEN UND MEERESMIKROALGEN
ESTER MÉTHYLIQUE D'ACIDE GRAS (BIODIESEL) DIGNE DES MOTEURS OBTENU À L'AIDE DE NAPPES DE MICROALGUES MARINES NATURELLES ET DE MICROALGUES MARINES CULTIVÉES DANS DES CUVETTES SALÉES À CIEL OUVERT AVEC VALORISATION PAR DES CO-PRODUITS

(30) Priority: 26.05.2011 IN DE15072011
(43) Date of publication of application: 16.04.2014
(73) Proprietor: Council of Scientific & Industrial Research, New Delhi 110 001 (IN)
(72) Inventor: MISHRA, Sandhya, Chandrika, Prasad, Bhavnagar 364021 Gujarat (IN); GHOSH, Pushpito, Kumar, Bhavnagar 364021 Gujarat (IN); GANDHI, Mahesh, Ramniklal, Bhavnagar 364021 Gujarat (IN); BHATTACHARYA, Sourish, Bhavnagar 364021 Gujarat (IN); MAITI, Subarna, Bhavnagar 364021 Gujarat (IN); UPADHYAY, Sumesh, Chandra, Bhavnagar 364021 Gujarat (IN); GHOSH, Arup, Bhavnagar 364002 Gujarat (IN); PRASAD, Rachapudi, Badari, Narayana, Hyderabad 500007 (IN); KANJILAL, Sanjit, Hyderabad 500007 (IN); MISHRA, Sanjiv, Kumar, Bhavnagar 364021 Gujarat (IN); SHRIVASTAV, Anupama, Vijaykumar, Bhavnagar 364021 Gujarat (IN); PANCHA, Imran, Bhavnagar 364021 Gujarat (IN); PALIWAL, Chetan, Bhavnagar 364021 Gujarat (IN); GHOSH, Tonmoy, Bhavnagar 364021 Gujarat (IN); MAURYA, Rahul, Kumar, Bhavnagar 364021 Gujarat (IN); JAIN, Deepti, Bhavnagar 364021 Gujarat (IN); PATIDAR, Shailesh, Kumar, Bhavnagar 364021 Gujarat (IN); SAHU, Abhishek, Bhavnagar 364021 Gujarat (IN); BOSAMIYA, Hetal, Bhavnagar 364021 Gujarat (IN); ZALA, Krushnadevsinh, Bhavnagar 364021 Gujarat (IN)
(74) Representative: Robertson, James Alexander
(86) International application number: PCT/IN2012/000372
(87) International publication number: WO 2012/160577

(56) References cited:
- WO-A1-2011/027353
- WO-A1-2012/038978
- WO-A2-2008/120223
- US-A1- 2009 211 150
- US-A1- 2009 298 159
- BOONPRAB K ET AL: "BIODIESEL FROM FRESH WATER ALGAE, CLADOPHORA GLOMERATA", PHYCOLOGIA, BLACKWELL SCIENTIFIC PUBL., OXFORD, GB, vol. 48, no. 4, Suppl. S, 2 August 2009 (2009-08-02), page 11, XP009149337, ISSN: 0031-8884
- XU H ET AL: "High quality biodiesel production from a microalga Chlorella protothecoides by heterotrophic growth in fermenters", JOURNAL OF BIOTECHNOLOGY, ELSEVIER SCIENCE PUBLISHERS, AMSTERDAM, NL, vol. 126, no. 4, 1 December 2006 (2006-12-01), pages 499-507, XP024956582, ISSN: 0168-1656, DOI: 10.1016/J.JBIOTEC.2006.05.002 [retrieved on 2006-12-01]
- MARCELO G MONTES D OCA ET AL: "Production of FAMEs from several microalgal lipidic extracts and direct transesterification of the", BIOMASS AND BIOENERGY, PERGAMON, AMSTERDAM, NL, vol. 35, no. 4, 21 December 2010 (2010-12-21), pages 1533-1538, XP028167175, ISSN: 0961-9534, DOI: 10.1016/J.BIOMBIOE.2010.12.047 [retrieved on 2010-12-28]
- YANGMIN GONG ET AL: "Biodiesel production with microalgae as feedstock: from strains to biodiesel", BIOTECHNOLOGY LETTERS, SPRINGER NETHERLANDS, DORDRECHT, vol. 33, no. 7, 5 March 2011 (2011-03-05), pages 1269-1284, XP019916058, ISSN: 1573-6776, DOI: 10.1007/S10529-011-0574-Z
- BRENNAN L ET AL: "Biofuels from microalgaeA review of technologies for production, processing, and extractions of biofuels and co-products", RENEWABLE AND SUSTAINABLE ENERGY REVIEWS, ELSEVIERS SCIENCE, NEW YORK, NY, US, vol. 14, no. 2, 1 February 2010 (2010-02-01), pages 557-577, XP026811476, ISSN: 1364-0321 [retrieved on 2009-10-29]

## Description

### FIELD OF THE INVENTION

The present invention relates to utilizing naturally occurring lipid-bearing microalgal mats collected from the west coast of India for the production of fatty acid methyl ester (FAME) and demonstrating the suitability of the latter for the running of a regular vehicle. The invention also relates to raising readily harvestable lipid-bearing microalgal biomass (raised from *Chlorella variabilis* Accession No. PTA-12198) in solar salt pans and its further conversion into FAME which too is engine worthy.

### BACKGROUND OF THE INVENTION

Reference may be made to the article by Daemon Fairless, Biofuel: The little shrub that could-may be. Nature (2007) 449, 652-655 and Laurent Lardon et al, Life-Cycle Assessment of Biodiesel Production from Microalgae, Environmental Science & Technology (2009) 43:17, 6475- 6481 wherein, the complex issue of food versus fuel is highlighted and the need for such biomass sources which would not interfere with food production is emphasised.

Reference may also be made by D.H. Lee, Algal biodiesel economy and competition among biofuels, Bioresource Technology (2011) 102, 43-49, to the scarcity of arable land and the need for alternative locations for generating biomass useful for fuel production, in which microalgae cultivation does not require large areas of arable land. Furthermore, cultivation sites can either be in desert areas or in shallow coastal waters.

Reference may be made to Ghosh et al US Patent 7666234, wherein, the utility of marginal lands for engine worthy biodiesel production is disclosed. The biomass in question is of terrestrial origin.

Reference may be made to the paper entitled "Biodiesel production by simultaneous extraction and conversion of total lipids from microalgae, cyanobacteria, and wild mixed-cultures" (B. D. Wahlen et al. Bioresource Technol., 2011, 102, 2724-2730).

Reference may be made to the articles by Doan et al, Screening of marine microalgae for biodiesel feedstock, Biomass and Bioenergy (2011) 35:7; 2534-2544 and Matsunaga et al. Characterization of marine microalga, Scenedesmus sp. strain JPCC GA0024 toward biofuel production. Biotechnology Letters (2009) 31: 1367-1372 wherein, marine microalgal species are reported to contain lipids which could serve as a source of biodiesel.

Reference may be made to US patent 7977076; Nasrin Moazami et al. Biomass and lipid productivities of marine microalgae isolated from the Persian Gulf and the Qeshm Island.

Reference may also be made to the article by Brennan et al, Biofuels from microalgae: A review of technologies for production, processing, and extractions of biofuels and co-products. Renewable and Sustainable Energy Reviews (2010) 14: 557-577 which, states that microalgae are normally cultured for low volume high value products requiring limited land area or where the biomass can be raised indoors in photo-bioreactors.

Reference may be made to the articles by Hankamer et al, Photosynthetic biomass and H2 production by green algae: from bioengineering to bioreactor scale-up Physiologia Plantarum (2007) 131: 10-21 and Wang et al, CO2 bio-mitigation using microalgae. Applied Microbiology and Biotechnology (2008) 79: 5; 707-718; wherein, the importance of photosynthetic production of microalgal biomass for biofuel production is emphasised.

Reference may be made to the article by Douskova et al, Simultaneous flue gas bioremediation and reduction of microalgal biomass production costs, Applied Microbiology & Biotechnology (2009), 82:179-185 wherein, it is claimed that biomass productivity of microalgae can be raised through use of flue gas as rich source of CO₂.

Reference may be made to any number of articles like Griffiths et al Lipid productivity as a key characteristic for choosing algal species for biodiesel production. Journal of Applied Phycology 2009, 21:493-507 and Report prepared for Sustainable Energy Ireland by Tom Bruton et al. A Review of the Potential of Marine Algae as a Source of Biofuel in Ireland (2009) which mention the importance of marine microalgae as source of biodiesel but, do not disclose where or how such cultivation will be taken up on a scale relevant to large scale biodiesel production.

Reference may be made to a review by Pittman et al The potential of sustainable algal biofuel production using wastewater resources, Bioresource Technology (2011) 102, 17-25. However, no mention is made therein of their utility for biodiesel production, besides *Microspora sp.* of mix culture from agricultural waste was neither determined nor used for lipid.

Reference may be made to the work by Syed Zahir Shah & Habib-ur-Rehman Khattak (Some Green Algae from Paddy Fields of Mathra (District Peshawar), Syed Zahir Shah & Habib-ur-Rehman Khattak, Department of Botany, Islamia College, Peshawar) disclosing the presence of *Microspora* sp. near Sind River as part of a study on biodiversity. No mention is made therein of any utility of the biomass.

Reference may be made to the paper entitled Cell division and wall structure in *Microspora* (Picketts-Heaps. New Phytologist, (1973) 72,347-355) wherein the cytology of *Microspora* sp. is discussed. It is stated therein that, such alga may occur in the form of a mat. It is further stated that the alga may or may not contain lipids. No reference is made to any attempt to utilize such mats for biodiesel preparation.

Reference may be made to the article by Mata et al, Microalgae for biodiesel production and other applications: A review. Renewable and Sustainable Energy Reviews (2010) 14: 217-232; this enumerates the various species of marine microalgae which are lipid bearing and, in that sense, of potential interest as source of biodiesel.

Reference may be also made to the article by Greenwell et al, placing microalgae on the biofuels priority list: a review of the technological challenges. Journal of the royal society interface (2010), 7: 703-726 which says that, different microalgae grow at different rates and whereas some have high oil content they are impractical to use for other reasons such as slow growth rate, difficulty of harvesting the biomass, etc.

Reference may be made to the US2009/0298159 A1 wherein a method is provided to produce biodiesel from algae using two-stage, autotrophic and heterotrophic, growth phases of *Chlorella variabilis* for biodiesel production which includes a sequence of procedures: cultivating photoautotrophic algae, concentrating the cells and then transferring them to a fermentor for heterotrophic cultivation. Organic carbon is added during the heterotrophic cultivation stage. It is evident that the process is conducted in closed systems and requires concentration of the cells which is energy intensive. Moreover, no mention is made of the characteristics of the biodiesel nor of any trials conducted on vehicles.

Reference may be made to the site www.treehugger.com/chevron-backs-solazyme-to-develop-algal-biodiesel-technology.html to the article entitled "Chevron Backs Solazyme to Develop Algal Biodiesel Technology" dated 2nd February 2008, wherein Solazyme is producing biodiesel from sugar sources through fermentation in the dark.

It will be evident from the prior art that no cost-effective process has been disclosed for production of fatty acid methyl esters from such cultured or harvested biomass obtained from naturally occurring mats of microalgae. The present invention seeks to overcome all of these basic limitations and to evolve a novel, simplified and cost-effective process for producing fatty acid methyl ester from microalgal mats along with value added by-products from the waste co-streams. The total carbon of the water at the site was found to decrease with each subsequent collection. It may be attributed to the frequent collections from the same site.

Reference may be made to the patent application WO 2011/027353 (PCT/IN2010/000192) by Ghosh et al which discloses the culturing of Chlorella marine microalgal species and the advantages of mixotrophic growth. No reference is made therein of biodiesel production from the biomass nor of its practical cultivation on large scale so that the biomass is perceived as a high volume, affordable feedstock.

Reference may by be to the article K. Boonprab, Biodiesel from fresh water algae, Cladophora Glomerata, Phycologia, (2009), vol. 48(49, Suppl. 2, page 11 wherein a process for producing fatty acid methyl esters via alkali transesterification using lipid obtained from Cladophora glomerata by Soxlet extraction is disclosed.

Reference may be made to the article by Greenwell et al, "Placing microalgae on the biofuels priority list: a review of the technological challenges.", Journal of the royal society interface (2010), 7: 703-726 and Chisti, Y et al; Biotechnology Advances (2003) which, brings out the difficulties of downstream processing of microalgal biomass and the high energy penalty as a result. Foremost among these is the difficulty of harvesting the biomass from highly dilute suspensions.

Reference may be made to the articles http://www.treehugger.com/renewable-energy/chevron-backs-solazyme-to-develop-algal-biodiesel-technology.html & http://www.treehugger.com/cars/solazyme-b100-algae-biodiesel-goes-on-the-road.html disclosing the running of a vehicle on B100 microalgal biodiesel. However, the article states that the biomass was raised under heterotrophic conditions using sugars as organic carbon source.

There is no report on the performance of any biodiesel obtained from naturally occurring marine microalgal mats or thermo- tolerant marine microalgal biomass raised under practical conditions of autotrophic growth in solar salt pans.

During the search for the potential micro algae along the west coast, our team came across few sites showing probability of getting some desired microalgae through images observed in Google earth software. One of the sites we came across was located at Longitude 70°54.959' E and Latitude 20°42.391 N (site 1), and another site located at Longitude 68° 59. 876' and Latitude 22°23.975' (site 2), India, it showed dense micro algal mat. On microscopic examination (morphology) of the mat, it revealed that the mat contained various algal species among which *Microspora sp.* was found to be dominant. Available method for biodiesel from algae is much energy consuming. Hence, attempts have been made to develop cost effective process to produce biodiesel from marine micro-algae.

Reference may be made to Bligh, E.G., Dyer, W.J., A rapid method for total lipid extraction and purification, Canadian Journal of Biochemistry and Physiology (1959). 37, 911-917.Lee et al. Comparison of several methods for effective lipid extraction from microalgae, Bioresource Technology 101 (2010) S75-S7,the method of extracting lipids from marine microalgal biomass which typically entails extraction with polar solvents and yields large amounts of phospholipids besides triglycerides, the former being undesirable for biodiesel production.

### OBJECTS OF THE INVENTION

The main object of the present invention is to utilize naturally occurring mats of marine micro algal consortium and auto-settling marine microalgae raised in solar salt pans as sources of engine worthy fatty acid methyl ester (biodiesel).

Another object is to identify *Cladophora* sp. (ATCC Accession No. PTA-12199) and *Microspora* sp. (ATCC Accession No. PTA-12197) as the dominant species in the mats of the marine microalgal consortium referred to above.

Another object is to artificially cultivate such floating mats in solar salt pans and/or to raise lipid content through application of stress conditions.

Another object is to utilize *Chlorella variabilis* (ATCC Accession No. PTA-12198) isolated from the west coast of India as the auto-settling and thermo-tolerant marine microalgal strain ideal for raising in solar salt pans under summer conditions in Gujarat, India.

Another object is to minimize the energy penalty associated with lipid isolation from the biomass.

Another object is to utilize readily harvestable biomass as a means to reduce the energy penalty.

Another object is to create the opportunity to raise marine microalgal biomass on large scale utilizing ca.0.1 million acres of surplus land available for solar salt production.

Another object is to minimise the input costs for cultivation by using seawater, cheap inorganic nutrients, and avoiding mechanical gadgets for agitation of the culture medium while still achieving a maximum daily growth rate 45 g (dry basis)/m²/day during summer months. Another object is to draw on basic practices followed in solar salt production such as gravity feeding and shifting of liquids from pan to pan.

Another object is to sundry the harvested biomass.

Another object is to extract lipid from sundried biomass using volatile non-polar solvents such as hexane to minimise the proportion of undesired lipids in the overall extracted lipid mass.

Another object is to optionally utilize fossil diesel for the extraction process where blended biodiesel is used.

Another object is to utilise solar thermal energy for the extraction and lipid isolation processes to maximise the energy output to input ratio.

Another object is to derive maximum value from the spent biomass.

Another object is to refine the raw oil through simple and cost effective means.

Another object is thereafter to draw on the known process of FAME production as disclosed in US Patent 7666234.

Another object is to demonstrate production of marine microalgal FAME with desired specifications of basic parameters such as viscosity, free fatty acid content, oxidation stability, free and total glycerol, phosphorous content, moisture content etc.

Another object is to demonstrate the running of a standard vehicle with B20 biodiesel produced from the marine microalgal mats and B100 biodiesel produced from *Chlorella variabilis* (ATCC Accession No. PTA-12198)

Another object is to utilise the by-product crude glycerol stream for accelerated growth and higher lipid content of the cultured microalgae as disclosed in the prior art.

### SUMMARY OF THE INVENTION

The present invention provide Fatty acid methyl ester (FAME) for use as biodiesel, the esters being produced from naturally floating marine microalgal mats or thick layers of settled marine microalgae being formed during cultivation in solar salt pans or mixture thereof.

An aspect of the present invention provides an integrated process for the preparation of engine worthy fatty acid methyl ester (biodiesel) from naturally harvested floating mats with consortium of *Microspora* ATCC Accession No. PTA-12197 and *Cladophora* ATCC Accession no. PTA-12199 and cultured microalgal mat along with the mass cultivated selected thermo-tolerant strain (*Chlorella variabilis* ATCC Accession No. PTA-12198) of microalgae and utilization of the by-products from the microalgal mass as well as the by-products from the fatty acid methyl esters and the said process comprising the steps of:
a) collection of the microalgal mats consortium from different sites and washing to remove the adhering sand and dirt particles ii) identification of the microalgal species present in the mat and culturing of the mat under laboratory conditions using sea water and the CSMCRI Experimental Salt Farm (ESF) sea salt (5°Be¢) and to simulate the natural conditions in the tanks for further proliferation.
b) Isolation of oil bearing microalgal species (*Microspora, Rhizoclonium, Spirulina, Chlorella, Cladophora, Diatoms, Oscillatoria spp., etc.)* from the mat.
c) outdoor mass cultivation of the oil bearing microalgal species in experimental salt farm in 18m² and 90m² tanks and auto- settling of the biomass which facilitates easy harvesting and recycling of the supernatant as the nutrient for the inoculum of next batch.
d) drying of the microalgal mats followed by grinding of the dried microalgal mats into fine powder.
e) efficient extraction of oil from the biomass which includes pre-treatment of the biomass using ball mill/steam followed by non-polar solvent extraction / Soxhlet, etc. and recycling the solvent.
f) refining of the raw oil
g) following known transesterification processes to prepare engine worthy biodiesel as in the prior art.
h) utilization of the biodiesel co-product streams to raise algal productivity through Mixotrophic growth as disclosed in the prior art (WO2012/038978 (Patent application PCT/IN2011/000655)) and/or for production of other useful materials such as biodegradable biopolymer (WIPO Patent Application WO/2011/027353).
i) utilization of the spent biomass and wastewater generated during synthesis of biodiesel for biogas production.
j) utilization of the spent biomass as an aqua- feed and as a biofertilizer.
k) utilization of the residual biomass and deoiled cake for preparation of briquettes.
l) extraction of carotenoids from the deoiled residual biomass.
m) formulation of blends B20 and B100 suitable for running vehicle (Chevrolet Tavera) with full load without any engine modifications.

In an embodiment of the present invention is disclosed a process for the production of engine worthy fatty acid methyl ester for use as biodiesel, the process comprises the steps of:
(i) collecting naturally occurring microalgal mats having consortium of *Microspora sp. and Cladophora sp.* and cultivated *Chlorella variabilis* to obtain algal biomass;
(ii) sun drying the biomass to residual moisture level of 5-10%;
(iii) pre-treating the biomass of step (ii) by steam blast or osmotic shock to disrupt the cell wall;
(iv) extracting lipid from algal biomass of step (iii) using hexane as a solvent or optionally with diesel where the fuel is to be used in blend form to obtain raw oil;
(v) stripping off the hexane and treating resultant raw oil with fullers earth or optionally treating the extract of step (ii) directly with fullers earth to remove phospholipids, pigments and other impurities;
(vi) filtering to remove suspended solids and treating the oil extract of step (v) further to reduce free fatty acid (FFA) content, if required to obtain refined oil;
(vii) undertaking alkali-catalyzed transesterification of refined oil of step (vi), separating the FAME, and purifying it further to obtain engine worthy FAME.

In another embodiment of the present invention is disclosed a process for preparing FAME, wherein FAME obtained from Microspora sp.is. having composition as analyzed by GC-MS comprising 9.92% of 16:0 fatty acid, 2.44% of 18:0 fatty acid, 28.27% of 18:1 fatty acid, 59.37% of 18:2 fatty acid, and 5-30 ppm of BHT antioxidant.

In yet another embodiment of the present invention is disclosed a process, wherein FAME obtained from *Chlorella variabilis is* having composition as analyzed by GC-MS comprising 6.9% of 16:0 fatty acid, 3.1% of 18:0 fatty acid; 32.6% of 18:1 fatty acid, and 57.3% of 18:2 fatty acid, and 5-30 ppm of BHT antioxidant

In one embodiment of the present invention, the present invention provides an integrated process for the preparation of engine worthy fatty acid methyl ester (biodiesel) from nature and cultured microalgal mat along with the mass cultivated selected strain of microalgae and utilization of the by-products from the microalgal mass as well as the by-products from the fatty acid methyl esters.

In another embodiment of the present invention, the microalgal mat is a consortium of different microalgal species with *Microspora* and *Cladophora* spp. ATCC Accession No. PTA-12199 as the dominant species.

In yet another embodiment of the present invention, sea water with essential micronutrients/CSMCRI-ESF salt is used for the outdoor mass cultivation of the microalgae.

In yet other embodiment of the present invention, the oil extraction was done using solvents selected from the group consisting of hexane, chloroform, methanol, acetone, tetrahydrofuran, diethyl ether; preferably hexane, chloroform and methanol.

In a yet another embodiment of the present invention, the biodiesel co-product streams are used for production of PHA-biopolymers, biogas, gasification, fertilizer, aqua feed, carotenoids and for the preparation of briquettes.

### BRIEF DESCRIPTION OF THE INVENTION

A few sites showing the probability of getting some desired microalgae were selected through images observed in Google Earth software. One of the sites was located at Longitude 70° 54.959' E and Latitude 20° 42.391 N (site 1), and another site located at Longitude 68° 59. 876' and Latitude 22°23.975' (site 2), India. It showed dense floating microalgal mat. On microscopic examination (morphology) of the mat, it revealed that the mat contained various microalgal species among which *Microspora* sp. and *Cladophora* sp. ATCC Accession No. PTA-12199 was found to be dominant. Available methods for biodiesel from algae is much energy consuming. Hence, attempts have been made to develop a cost effective process to produce biodiesel from a consortium in marine micro-algal mat as well as the isolated and mass cultivated strain of *Chlorella variabilis* ATCC Accession no. PTA-12198.

Utilization of the microalgal mats containing *Microspora* and *Cladophora* spp. ATCC Accession No. PTA-12199 (dominant from site 1 and 2 respectively) for biodiesel production with an integrated process is unique. The natural mat is widely spread and found to regenerate within a few weeks after it has been harvested. Besides, it was observed to regenerate at other experimental sites too. Mat of consortium with dominant *Microspora* and *Cladophora* spp. ATCC Accession No. PTA-12199 was found to survive and grow in a variable range of environmental parameters.

### Novel Features of the invention:

The main inventive steps are the following:
- Recognising that the harvesting of microalgae is energy intensive and taking advantage of the ingenuity of nature in creating lipid bearing floating marine microalgal mats such as those having *Microspora sp.* (ATCC Accession No. PTA-12197) and *Cladophora sp.* (PTA 12199) as dominant species which can simply be skimmed off from the water and processed further.
- Recognising that whereas certain conditions in nature are conducive for natural growth of marine microalgal mats, this kind of natural growth is confined to certain specific periods such as the few months following the monsoon and their occurrence being sparse during other months such as the summer months. Further recognising after much labour that it is not always easy to simulate those natural conditions and that other varieties of marine microalgae may be better suited for cultivation.
- Recognising that if marine microalgae need to be cultivated to supplement natural stocks and also for year round harvest, then a useful approach is to harvest from nature in sustainable manner during the post monsoon period and thereafter take recourse to cultivation of marine microalgae in solar salt pans which may provide an ideal opportunity for large scale cultivation given that as much as 50% of available land for salt production is lying idle in India and these can be put to productive use.
- Identifying *Chlorella variabilis* from Indian waters (ATCC accession number PTA 12198) as a thermo-tolerant variety which grows well under autotrophic conditions during summer months with maximum observed dry biomass productivity of 45 g/m²/day with minimum nutrition inputs and also avoiding energy intensive measures such as continuous agitation as adopted in raceway ponds.
- Also observing that under prevailing hot conditions during summer months, the selected strain of *Chlorella* grows and settles down thereby forming a thick layer at the bottom which is readily harvesting after draining out the supernatant which serves as inoculum for the subsequent batch of cultivation.
- Recognising that it may be feasible to grow the *Chlorella* even in non-summer months by use of solar reflectors placed in salt pans which enhance the radiation incident on the pan and consequently raise the temperature and incident photon radiation, both of which have a positive effect on biomass productivity and lipid yield.
- Recognising that lipids are both of polar and non-polar type and that it is the latter which is required for engine worthy biodiesel and thereafter sacrificing high lipid yield for desirable lipid fraction and extracting the biomass with non-polar solvents such as hexane and diesel instead of the conventionally used polar solvents such as methanol/chloroform.
- Recognising further that although the use of non-polar solvent for extraction gives a cleaner oil fraction, the dislodging of the microalgal cell wall is less efficient; accordingly, giving steam blast as a pre-treatment prior to the extraction; recognising further that such processes would be more energy efficient if they are solar driven.
- Using simple means such as filtration over Fuller's Earth to refine the raw oil and thereby using conventional methods of base-catalyzed transesterification such as those disclosed in the prior art for production of engine worthy fatty acid methyl ester.
- Producing methyl esters both from natural harvest and cultivated marine microalgae which were of a quality suitable for use in regular diesel vehicle.
- Utilising the by-product glycerol stream from marine microalgal biodiesel production as nutrient to improve the biomass productivity and lipid content under mixotrophic conditions as disclosed in the prior art.
- Utilising residual biomass after lipid extraction for diverse applications such as manure, source of carotenoids, aqua feed, source of energy, etc.
- Domestic sewage and crude sea salts low in NaCl but high in nutrients such as calcium, magnesium, and sulphate are used as media supplement for growth of microalgal mat and isolated marine microalgal cultures.

### Example 1

With the help of Google Maps(RTM) a search was undertaken of green patches in coastal waters which may help us to identify possible floating microalgal mats. Some of the prominent green patches found were in the coastal regions of Goa (Madkai; 15°41.0616' N, 73°95.6227' E), Kerala (Vellanathuruthu Road; 9°01.6659' N, 76°52.5022' E), West Bengal (Port Canning, 22° 31.5577'N, 88° 67.3307' E;, Dongajora, 22° 13.2696'N, 88° 60.2676' E; Haldia refinery, 22° 04.9408' N, 88° 07.308' E), Diu (Nagoa road side, 70° 54.959' E, 20° 42.391 N) and Gujarat (Okha, 68° 59. 876', 22°23.975'). Ground truthing was undertaken of the sites identified in Diu and Gujarat and indeed green coloured floating mats of microalgae were found.

### Example 2

The mats of Example 1 were collected and observed under the Microscope (Carl Zeiss Axio Imager at 40x) for taxonomic identification. Both mats revealed a consortium of microalgae which were dominated by the *Chlorophycae* family. The one collected from 70° 54.959' E, 20° 42.391 N had *Microspora* as the dominant form whereas the one collected from 68° 59. 876', 22°23.975' was dominated by *Cladophora.* Isolation of associated species of the consortium was carried out by using serial dilution method. The algal mat was washed with distilled water to remove the adhering dirt and impurities and was further subjected to centrifugation. The supernatant was collected and inoculated in 24 well tissue culture plates with different culture media (BG-11, BBM, Zarrouk's, ASN-III, etc). The serial dilution was carried out using 1:10 dilution. The tissue culture plates were kept in artificial light (300 lux) in 12hr light and dark cycle at 25°C. After visible growth, the enriched culture were streaked on solid 1% Agarose plates. The Petri plates were incubated under artificial light (300 lux) in 12hr light and dark cycle at 25°C.The isolated culture was inoculated aseptically in liquid medium and kept in artificial light (300 lux) in 12hr light and dark cycle at 25°C. The mats from the above two locations were lyophilized and sent to American Type Culture Collection Centre (ATCC) for viability testing prior to allotment of accession numbers. One of the mats having *Cladophora* as the dominant lipid-bearing strain has been given ATCC Accession No. PTA-12199 while the viability testing of the other mat having *Microspora* as the dominant lipid-bearing strain is underway.

### Example 3

Naturally occurring marine microalgal mats were skimmed off from the microalgae dominated site Longitude 70° 54.959' E and Latitude 20° 42.391 N. The site was visited after 3-4 weeks on regular basis to study re-growth of the mats. During Summer season, the biomass productivity was 22.22 g/m²/day and total lipid content was 10%; during monsoon, the biomass productivity was 6.03 g/m²/day and total lipid content was 9.61% and during winter biomass productivity of 16 g/m²/day and total lipid content of 12.85% was achieved. This example teaches us that it is feasible to harvest microalgal mats from nature in sustainable manner.

### Example 4

The effect of elevated solar radiation on biomass productivity of ATCC- *Chlorella variabilis* was studied during winter (Air temp. 25-30 °C) in open tanks. Two tanks having 1.51 m² area and depth of 0.3 m containing 200 L sea water medium were inoculated with 10 % inoculum of *Chlorella* culture (OD₅₄₀ₙₘ= 1.65) The dry biomass yield after 14 days was 5.03 g/l with reflectors whereas the yield was 4.07 g/l in the control tank. This example teaches us the beneficial effect that solar reflectors can have on the cultivation process, especially when the ambient temperature is less than optimum.

### Example 5

Mass cultivation of *Chlorella variabilis* ATCC Accession No. PTA-12198 was carried out at the Institute's experimental salt farm (21° 47.488' N 72° 07.316' E Elevation: 28 ft.). The cultivation was carried out during the months of March-June. The outdoor temperature during the cultivation was 45±3 °C. The culture needed for this purpose was first grown in two tanks with an area of 18 m² each which were first used as inoculum tanks. The tanks were monitored regularly by measuring the pH, OD at 540 nm and biomass yield. After a cell concentration of 5g/l (wet basis) was reached, the culture was used to inoculate 7 more tanks with an area of 18 m² each and 3 tanks with an area of 90 m² each. pH, OD at 540 nm, biomass yield and environmental parameters were regularly monitored. The tanks were agitated manually (thrice a day) using a hollow pipe tied with strings at its ends up to 18 days. After 20 days of cultivation, it was observed that the biomass settled automatically forming a thick layer at the bottom of the tanks. Data on biomass productivity is given in the table below.

| Pond | Volume (L) | Dry Biomass (kg) | Biomass Productivity (g/m²/d) |
|---|---|---|---|
| P2 | 5000 | 11.75 | 32.64 |
| P3 | 5000 | 9.8 | 27.22 |
| P4 | 5000 | 8.4 | 23.33 |
| P5 | 5000 | 10.08 | 28 |
| P10 | 5000 | 7.5 | 20.83 |
| P11 | 5000 | 13.95 | 38.75 |
| P12 | 5000 | 4.2 | 11.67 |
| XL1 | 20000 | 52.1 | 28.94 |
| XL2 | 20000 | 73.8 | 41 |
| XL3 | 20000 | 56.2 | 31.22 |

The supernatant from each tank was transferred into an empty tank and the settled biomass was collected and sun dried. This example teaches us the feasibility of cultivating *Chlorella variabilis* (ATCC Accession No. PTA-12198) in solar salt pans

### Example 6

The experiments of Example 5 were repeated in two additional pans. 25 kg sodium bicarbonate, 6 kg sodium nitrate and 62.5 g of ferrous sulphate were added into 5000 L of the seawater culture medium. The biomass productivity was found to increase as can be seen from the table below.

| Pond | Volume (L) | Dry Biomass (kg) | Biomass Productivity (g/m²/d) |
|---|---|---|---|
| P6 | 5000 | 16.2 | 45 |
| P8 | 5000 | 16.4 | 45.56 |

This example teaches us that biomass productivity can be enhanced through addition of certain critical nutrients into the seawater medium.

### Example 7

Hexane extraction of lipid was conducted on the microalgal mats harvested from nature. Hexane was used as solvent. The data are provided in the table below. As can be seen, the lipid content varied from 5-16%.

| **Batch No.** | **Collection month of biomass** | **Sun dried Crude biomass (Kg)** | **Sand % in crude biomass** | **Oil obtained through Hexane extraction (Kg)** | **Dil yield on and-free bas (%)** |
|---|---|---|---|---|---|
| MM/NPL/2010/Batch 2 | Sep-10 | 25.0 | 35.0 | 1.63 | 9.07 |
| MM/NPL/2010/Batch 3 | Nov-10 | 34.8 | 40.0 | 1.79 | 8.65 |
| MM/NPL/2010/Batch 4 | Nov-10 | 15.6 | 40.0 | 0.68 | 7.26 |
| MM/NPL/2010/Batch 5 | Nov-10 | 46.0 | 40.0 | 1.44 | 5.22 |
| MM/NPL/2010/Batch 6 | Dec-10 | 72.1 | 50.0 | 2.03 | 5.63 |
| MM/NPL/2011/Batch 7 | Apr-11 | 12.1 | 20.0 | 1.58 | 16.32 |
| **Note:** For MM/NPL/2010/Batch 2,3,4 collection from Site 1 NRS, Diu; for MM/NPL/2010/Batch 5, 6, 7 collection from Site 2 NPI, Diu. | | | | | |

### Example 8

The study of Example 7 was repeated with *Chlorella variabilis* biomass of Examples 5 and 6. The data are provided in the table below. This example teaches us that cultivated biomass gives a more consistent oil yield.

| **Batch** | Month of Harvest | Dry Weight (Kg) | Moisture (%) | Raw Oil Weight (Kg) | Yield (%) |
|---|---|---|---|---|---|
| ESF/NPL/Batch 1 | Jun-11 | 209.9 | 8.0 | 21.45 | 11.11 |
| ESF/NPL/Batch 2 | Jun-11 | 53.0 | 7.8 | 5.45 | 11.15 |
| ESF/NPL/Batch 3 | Jun-11 | 48.0 | 7.1 | 5 | 11.21 |

### Example 9

The Table below gives relevant data pertaining to the fatty acid composition of the raw oils of Examples 7 and 8 as analysed by GC-MS above.

| **Fatty acid** | **Composition (Wt %)** | |
|---|---|---|
| | | **Example 8** |
| 14:0 | 0.6 | 0.4 |
| 16:0 | 9.4 | 12.1 |
| 16:1 | 0.7 | 1.0 |
| 16:2 | - | 1.0 |
| 18:0 | 3.7 | 4.2 |
| 18:1 | 33.2 | 29.4 |
| 18:2 | 50.4 | 45.7 |
| 18:3(ALA) | - | 4.8 |
| 20:0 | 0.7 | - |
| 22:0 | 1.3 | 1.4 |

### Example 10

18.738 kg of oil obtained in batch 1 of Example 8 was taken in a stainless steel vessel and was heated to 90°C. 1.8 kg of Fullers earth was added to it. The oil was filtered to obtain 15.916 kg of clarified oil. The clarified oil was analysed for its FFA content and was found to contain 0.6% FFA. 13 g of NaOH was taken and dissolved in 65 ml of water. The alkali solution so prepared was added into the clarified oil and stirred for 15 minutes. It was filtered to remove soap. The filtrate clear oil weighed 15.210 kg. The refined oil was transesterified using 2.92 kg (3.756L) of methanol and 399.24 g of KOH. The contents were stirred for 90 minutes at ambient temperature and allowed to stand for 60 minutes. The glycerol layer containing excess alcohol and KOH was separated; the weight of the glycerol layer was 4 kg. The biodiesel layer was washed with 682 g of glycerol and allowed to settle for 60 minutes. The glycerol wash weighed 687 gm. The biodiesel layer was then washed with 1L of water till pH reached 7. It was dried by heating the content at 110°C. 13.35 kg of Biodiesel so obtained was analysed for free glycerol, total glycerol, moisture, viscosity and density. The data are provided in the table below.

| o. | Name of Analysis | Result |
|---|---|---|
| 1 | Raw oil amount | 18.738 kg |
| 2 | Oil obtained after refining | 15.210 kg |
| 4 | B100 FAME (fatty acid methyl ester) | 13.350 kg |
| 5. | Yield of B100 FAME with respect to crude oil | 71.25% (w/w) |
| 6. | Density at 25° C | 0.8704 g/cm³ |
| | at 40° C | 0.8591 g/cm³ |
| 7. | Yield of B100 FAME with respect to dry biomass | 7.92% (w/w) |
| | | 9.15% (v/w) |
| 8. | Viscosity | 4.8 cSt(40°C) |
| 9. | Total Glycerin | 0.15% |
| 10. | Free Glycerin | 0.02% |
| 11. | CFPP | -5° C |
| 12. | Phosphorous content | 5.1 ppm |
| 13. | Average mileage during 200 km TAVERA test run under full load | 11.2 km |
| 14 | Calorific Value | 9843 kcal/kg |

### Example 11

The study of Example 10 was also conducted with oil obtained from Batch 2 in Example 7. The data are provided in Table 6 below.

| No. | Analysis | Result |
|---|---|---|
| 1. | Total Glycerin of Biodiesel | 0.1014 % |
| 2. | Free Glycerin of Biodiesel | 0.0086 % |
| 3. | Density | 0.872g/ ml |
| 4. | Viscosity 40°C | 4.5 CS (at 40°C) |
| 5 | Calorific Value | 9879 kcal/kg |

### Example 12

The table below provides the fatty acid composition of the fatty acid methyl esters of Examples 10 and 11, respectively as analysed by GC-MS. It will be evident that the compositions are very clean.

| **Fatty acid** | **Composition (Wt %)** | |
|---|---|---|
| | | **Example 10** |
| 16:0 | 9.92 | 6.9 |
| 18:0 | 2.44 | 3.1 |
| 18:1 | 28.27 | 32.6 |
| 18:2 | 59.37 | 57.3 |

### Example 13

The data of Example 12 provided confidence that the marine microalgal biodiesels of Examples 10 and 11 may be engine worthy. B20 biodiesel prepared from the fatty acid methyl ester of Example 11 and B100 biodiesel of Example 10 were used directly in a regular TAVERA car without any modification whatsoever. No difficulty of any kind was seen in running of the vehicle and mileage similar to that of fossil diesel was estimated. A journalist had this to say about the running of the car on the B100 biodiesel of Example 11: *"This correspondent took a test ride in the Tavera that was flagged off by the minister. The experience was equivalent to that of any other diesel vehicle, accompanied by a monotonous hum by a diesel engine. The two-km drive around the Central Secretariat area was smooth and without any hiccups.* "(Dinsa Sachan, "Biodiesel from microalgae becomes a reality", Down to Earth, March 30, 2012; www.downtoearth.org.in).

### Example 14

Steam at 121 °C 15-psi pressure passed through the bed of 30 g of *Chlorella* biomass of calorific value 4590 kCal/Kg for 15 minutes. 10.1 g of this steam treated sample was taken in cellulosic thimble for lipid extraction in Automated Soxhlet of solvent capacity 150 ml for 4 hours with 100 ml of hexane at 80° C. The studies indicated that hexane extraction becomes more efficient after steam pretreatment and complete extraction required 10 hours compared to the 16 hours taken normally.

### Example 15

The extraction of carotenoids was done in a closed reaction vessel of 1 L capacity from 50 g deoiled microalgal biomass of Example 8. Extraction was conducted with 500 ml of 80 % (v/v) acetone and kept in a dark room at constant magnetic stirring of 200 rpm. After continuous magnetic stirring for 3 hours, the solution was evaporated and the acetone free extract was filtered via filter paper to obtain carotenoids as the retentate. The carotenoids obtained ranged from 2-4%.

### Example 16

### Biogas Production from Deoiled Residual Biomass of mat & Chlorella

The residual biomass from Examples 7 and 8 were used for biogas production. After the biogas generation, the biomass slurry, which has a lot of micronutrients, carbon and nitrogen, can be used as a biofertilizer. The residual biomass can also be used as an aqua feed; it has proteins, carbohydrates and essential micronutrients. Briquettes of the residual biomass can be prepared. Biogas production from waste microalgae biomass, after oil extraction, is potentially feasible and can considerably increase the energy yield from biomass. Therefore, it has been regarded as a necessary step in order to make biodiesel production from microalgae sustainable. (Torres and Jeison, 2010)

Residual deoiled biomass having calorific values of 1884.52 kcal/kg for deoiled cake of Example 7 and 1679.00, Kcal/kg for deoiled cake of Example 8 were taken. The digested slurry from the biogas plant was used as an inoculum for biogas production. The set was divided into three parts (1) Digester (5.0 L), (2) Glass holder bottle (1.0 L) and (3) Liquid displacement bottle (1.0 L). The digester was marked at 4.0 L capacity and its joints were made air tight by applying silicon tape and vacuum grease. Gas holder bottle of 1.0 L capacity was filled up to its mark with 1.0 L of a colour reagent. A graduated scale was pasted on it to measure the gas production accurately. The biogas experiment was conducted in continuous and batch mode for each Test (residual biomass) and one control digester each for the continuous and batch processes. The Hydraulic Retention time (HRT) for this experiment was 30 days and the feeding substrate was 5%; for continuous digester 134 ml (4.0 L/30 days=0.1333 or 134 ml) sample was replaced with 134 ml test [as 5% (6.7g) biomass + 67 ml slurry + 67 ml t/w] daily through feeding tube while in batch digester 200 g biomass was added directly (5% for 4.0 L). A diluted activation solution at a ratio of 1:10 (multivitamin tablet and cysteine hydrochloride) was used to induce the growth of micro flora for biogas production and maintaining the anaerobic conditions. The resulting effluent slurry was analysed every day for parameters like total solids, total volatile solids, pH, electrical conductivity, total organic carbon, available nitrogen and available phosphorus. The total biogas production was also measured every day. The average daily biogas production in digester of deoiled biomass of Example 7 and 8 were 426.26 and 446.02 ml d⁻¹, respectively, for the batch process and 270.51 and 473.15 ml d⁻¹ for the continuous process.

### Example 17

The microalgal biodiesel by-product containing crude glycerol was utilized as a nutrient source for Mixotrophic, and heterotrophic growth of *Chlorella variabilis,* where all flasks containing 100 ml. sea water medium with variation of Algal Biodiesel waste residue (ABWR) for Mixotrophic growth at room temperature. After inoculation the OD is 0.5 at 540 nm. After 8 days biomass productivity was observed to be maximum in 5g/L of ABWR (Mixotrophic). This example teaches us the utility of the crude glycerol stream in raising the biomass productivity.

### Example 18

The spent microalgal biomass is used as biofertilizer to promote growth and can substitute chemical fertilizers. The NPK content is 1.2: 0.03: 0.6 (%) for *Cladophora,* 1.4: 0.01: 1.1 (%) for *Microspora* and 2.19: 0.01: 1.0 (%) for *Chlorella.* Experiments were conducted in two plots for Maize crop with 6 lanes for control (K₂O) and 4 lanes each for *Cladophora, Microspora* and *Chlorella* on equivalent nutrient (K₂O) basis. The plant height, number of leaves per plant, numbers of cobs per plant, length and width of cobs and chlorophyll index were measured after eight weeks of growth. The results show that *Chlorella* gave the best results with an average plant height of 167.8±7.34 cm, 14.8±0.583 leaves per plant, 2 cobs per plant, 32.0±0.84 cm cob length, 7.24±0.24 cm cob width and 49.31±0.03 chlorophyll index (Opti-Sciences CCM-200, USA) as compared to control (chemical fertilizer K₂O) 158.4±2.79 cm plant height, 13.6±0.4 leaves per plant, 1.6±0.25 cobs per plant, 28.6±0.75 cm cob length, 7.24±0.24 cm cob width and 40.25±1.97 chlorophyll index. An increase of 16.43% in yield was observed when *Chlorella* was used as a biofertilizer instead of K₂O (control). This example teaches us a further utility of the deoiled cake.

### Example 19

The deoiled cake of Example 8 had calorific value of 1765.91kcal/kg. The algae was mixed with 10% by weight of wet cow dung , converted to hand- made briquettes of diameter 4 cm and depth 2 cm and were subjected to open sun drying. 30 kg of such dried biomass was then charged into the biomass gasifier of 15Kg/hr installed at the Institute's ESF premises. After about 10 minutes of gasifier operation, the combustible component of the producer gas was noted using an online gas analyser. The gas burnt with a yellow flame. This example teaches us that the deoiled cake can also be used in biomass gassifier.

| Time | Carbon monoxide | Methane | Hydrogen |
|---|---|---|---|
| 4.15 P.M | 0.30 | 0.23 | 2.52 |
| 4.20 P.M | 0.33 | 0.25 | 2.67 |
| 4.25 P.M | 0.33 | 0.30 | 3.13 |
| 4.30 P.M | 0.35 | 0.32 | 3.79 |

Advantages of the present invention are:
- The present invention provides a low cost option for generating marine microalgal biomass by harvesting naturally occurring mats of such algae in sustainable manner.
- The invention also has the advantage that a thermo-tolerant *Chlorella sp.* is found to be cultivable in open solar salt pans even in hot summer conditions with high biomass productivity and good lipid content.
- The invention has the further advantage that minimum nutrient and energy inputs are required for cultivation of the biomass.
- The invention also has the advantage that the biomass is readily harvestable.
- The invention has the further advantage that only the useful portion of lipids suitable for biodiesel production is selectively extracted with the help of non-polar solvent.
- The invention has the further advantage that simple methods are used to refine the raw oil obtainable with non-polar solvent extraction and thereafter the oil is readily processed into high quality biodiesel by known methods.
- The invention has the further advantage of demonstrating that such methyl ester obtained from marine microalgal sources can be utilised even under neat condition (B100) to run a regular diesel vehicle without any engine modification.
- The invention has an additional advantage of demonstrating the direct utility and/or value addition of certain co-product streams.

## Claims

1. A process for the production of engine worthy fatty acid methyl ester (FAME) for use as biodiesel, the process comprises the steps of:
(i) collecting naturally occurring marine microalgal mats selected from the group consisting of *Microspora sp.* and *Cladophora sp.* or cultivated *Chlorella variabilis* to obtain algal biomass;
(ii) sun drying the biomass to residual moisture level of 5-10%;
(iii) pre-treating the biomass of step (ii) by steam blast or osmotic shock to disrupt the cell wall;
(iv) extracting lipid from algal biomass of step (iii) using hexane as a solvent or optionally with diesel where the fuel is to be used in blend form to obtain raw oil;
(v) stripping off the hexane and treating resultant raw oil with fullers earth or optionally treating the extract of step (ii) directly with fullers earth to remove phospholipids, pigments and other impurities;
(vi) filtering to remove suspended solids and treating the oil extract of step (v) further to reduce free fatty acid (FFA) content, if required to obtain refined oil;
(vii) undertaking alkali-catalyzed transesterification of refined oil of step (vi), separating the FAME, and purifying it further to obtain engine worthy FAME.

2. The process as claimed in claim 1 wherein the lipid is extracted from marine microalgal mat comprising *Microspora sp.* (ATCC Accession Number PTA-12197) through extraction with hexane, the lipid having composition as analyzed by GC-MS 0.6% of 14:0 fatty acid, 9.4% of 16:0 fatty acid, 0.7 % of 16: 1 fatty acid, 3.7% of 18:0 fatty acid, 33.2% of 18:1 fatty acid, 50.4% of 18:2 fatty acid, 0.7% of 20:0 fatty acid, 1.3% of 22:0 fatty acid.

3. The process as claimed in claim 1 wherein lipid is extracted from marine microalgae *Chlorella variabilis* (ATCC Accession Number PTA 12198), through extraction with hexane, the lipid having composition as analyzed by GC-MS 0.4% of 14:0 fatty acid, 12.1% of 16:0 fatty acid, 1.0 % of 16: 1, 1.0% of 16:2 fatty acid, 4.2% of 18:0 fatty acid, 29.4% of 18: 1, 45.7% of 18:2 fatty acid, 4.8% of 18:3 fatty acid, 1.4% of 22:0.

4. The process as claimed in claim 1 wherein the lipid is extracted from marine microalgal mat comprising *Cladophora sp.* (ATCC Accession Number PTA 12199) through extraction with hexane, the lipid having composition as analyzed by GC-MS 0.9% of 14:0 fatty acid, 0.4% of 15:0 fatty acid, 21.5% of 16:0 fatty acid, 1% of 16:1 fatty acid, 2.9% of 18:0 fatty acid, 21.2% of 18:1 fatty acid, 22.3% of 18:2 fatty acid, 0.5% of 20:0 fatty acid, 16.3% of 20:1 fatty acid, 0.4% of 22:0 fatty acid, 11.4% of 22:1 fatty acid, 0.7% of 24:0 fatty acid, 0.6% of 24: 1 fatty acid.

5. The process as claimed in claim 2, wherein the lipid fraction obtained from *Microspora sp.* is refined and transesterified to obtain FAME having composition as analyzed by GC-MS comprising 9.92% of 16:0 fatty acid, 2.44% of 18:0 fatty acid, 28.27% of 18: 1 fatty acid, 59.37% of 18:2 fatty acid, and 5-30 ppm of BHT antioxidant; optionally wherein, the FAME is a clear yellow liquid having 0.872 gm/ml density, 4.5 cSt (at 40°C) viscosity, 0.1014% total glycerol and 0.0086% free glycerol and calorific value as measured by Standard calorimetric test is 9879 kcal/kg.

6. The process as claimed in claim 5-6 wherein the said FAME is used in a regular unmodified diesel vehicle as B20 blend under full load condition and complying emission requirements.

7. The process as claimed in claim 3, wherein the lipid fraction obtained from *Chlorella variabilis* (ATCC Accession Number PTA 12198) is refined and transesterified to obtain FAME having composition as analyzed by GC-MS comprising 6.9% of 16:0 fatty acid, 3.1% of 18:0 fatty acid; 32.6% of 18: 1 fatty acid, and 57.3% of 18:2 fatty acid, and 5-30 ppm of BHT antioxidant; optionally wherein, the said FAME is a clear mustard yellow liquid having density at 25°C and 40°C 0.8704 and 0.8591 g/cm³, respectively; viscosity at 40°C, 4.8 cSt; total glycerin, 0.15%; free glycerin , 0.02%; CFPP, moisture content, 0.029%; -5°C; Phosphorous, 5.1 ppm; oxidation stability, 0.43 years (25°C) and 0.12 year (40°C) and calorific value as measured by Standard calorimetric test is 9843kcal/kg,

8. The process as claimed in claims 8-9 wherein the said FAME is used in a regular unmodified diesel vehicle as B100 biodiesel under full load condition and complying emission requirements.

9. The process as claimed in claims 1-2, 5-7 and 11 wherein the lipid yield with hexane extraction for mats of *Microspora sp.* is in the range of 5.22-16.32%.

10. The process as claimed in claims 1, 3, 8-10 and 12-13 wherein the lipid yield with hexane extraction for the cultivated *Chlorella variabilis* (ATCC Accession Number PTA 12198) is in the range of 11.11-11.21 %.

11. The process as claimed in claims 1, 3, 8-10, 12-13 and 15 wherein growth rate and lipid yield of *Chlorella variabilis* is influenced by addition of 3-6 kg of sodium bicarbonate, 1-2 kg sodium nitrate, and 0.01-0.02 kg ferrous sulphate per 1000 L of the seawater culture medium.

12. The process as claimed in claims 1, 3, 8-10, 12-13 and 15-16 wherein crude glycerol from by-product streams of the FAME process is optionally added to enhance biomass productivity by 50-200%.

13. The process as claimed in claims 1-18, wherein residual biomass after solvent extraction of lipid is utilized in production of biofertilizer, aqua feed, source of carotenoids, and source of energy.

14. The process as claimed in claims 1-18, wherein co-product streams of crude glycerol is utilized for algal productivity through mixotrophic growth and/or for production of biodegradable biopolymer.

## Patentansprüche

1. Verfahren für die Erzeugung von motortauglichem Fettsäuremethylester (FAME) zur Verwendung als Biodiesel, wobei das Verfahren die Schritte umfasst:
(i) Sammeln von natürlich vorkommenden Meeresmikroalgenmatten, ausgewählt aus der Gruppe, bestehend aus *Microspora sp.* und *Cladophora sp.,* oder kultivierter *Chlorella variabilis***,** um Algenbiomasse zu erhalten;
(ii) Sonnentrocknen der Biomasse zu einem Restfeuchteniveau von 5-10%;
(iii) Vorbehandeln der Biomasse von Schritt (ii) durch Dampfgebläse oder osmotischen Schock, um die Zellwand zu zerreißen;
(iv) Extrahieren von Lipid aus der Algenbiomasse von Schritt (iii) unter Verwendung von Hexan als einem Lösungsmittel oder wahlweise mit Diesel, wo der Treibstoff in Mischform zu verwenden ist, um rohes Öl zu erhalten;
(v) Abstrippen des Hexans und Behandeln des resultierenden rohen Öls mit Fullererde oder wahlweise Behandeln des Extrakts von Schritt (ii) direkt mit Fullererde, um Phospholipide, Pigmente und andere Verunreinigungen zu entfernen;
(vi) Filtrieren, um suspendierte Feststoffe zu entfernen, und weiteres Behandeln des Ölextrakts von Schritt (v), um, falls erforderlich, um raffiniertes Öl zu erhalten, den Gehalt an freier Fettsäure (FFA) zu verringern;
(vii) Vornehmen von Alkali-katalysierter Umesterung des raffinierten Öls von Schritt (vi), Abtrennen des FAME und weiteres Reinigen desselben, um motortauglichen FAME zu erhalten.

2. Verfahren nach Anspruch 1, wobei das Lipid aus einer Meeresmikroalgenmatte, umfassend *Microspora sp.* (ATCC-Zugangsnummer PTA-12197), durch Extraktion mit Hexan extrahiert wird, wobei das Lipid eine Zusammensetzung, wie analysiert durch GC-MS, von 0,6% von 14:0-Fettsäure, 9,4% von 16:0-Fettsäure, 0,7% von 16:1-Fettsäure, 3,7% von 18:0-Fettsäure, 33,2% von 18:1-Fettsäure, 50,4% von 18:2-Fettsäure, 0,7% von 20:0-Fettsäure, 1,3% von 22:0-Fettsäure hat.

3. Verfahren nach Anspruch 1, wobei das Lipid aus den Meeresmikroalgen *Chlorella variabilis* (ATCC-Zugangsnummer PTA 12198) durch Extraktion mit Hexan extrahiert wird, wobei das Lipid eine Zusammensetzung, wie analysiert durch GC-MS, von 0,4% von 14:0-Fettsäure, 12,1% von 16:0-Fettsäure, 1,0 % von 16:1, 1,0% von 16:2-Fettsäure, 4,2% von 18:0-Fettsäure, 29,4% von 18:1, 45,7% von 18:2-Fettsäure, 4,8% von 18:3-Fettsäure, 1,4% von 22:0 hat.

4. Verfahren nach Anspruch 1 wobei das Lipid aus einer Meeresmikroalgenmatte, umfassend *Cladophora sp.* (ATCC-Zugangsnummer PTA 12199), durch Extraktion mit Hexan extrahiert wird, wobei das Lipid eine Zusammensetzung, wie analysiert durch GC-MS, von 0,9% von 14:0-Fettsäure, 0,4% von 15:0-Fettsäure, 21,5% von 16:0-Fettsäure, 1% von 16:1-Fettsäure, 2,9% von 18:0-Fettsäure, 21,2% von 18:1-Fettsäure, 22,3% von 18:2-Fettsäure, 0,5% von 20:0-Fettsäure, 16,3% von 20:1-Fettsäure, 0,4% von 22:0-Fettsäure, 11,4% von 22:1-Fettsäure, 0,7% von 24:0-Fettsäure, 0,6% von 24:1-Fettsäure hat.

5. Verfahren nach Anspruch 2, wobei die Lipidfraktion, erhalten aus *Microspora sp.,* raffiniert und umgeestert wird, um FAME mit einer Zusammensetzung, wie analysiert durch GC-MS, umfassend 9,92% von 16:0-Fettsäure, 2,44% von 18:0-Fettsäure, 28,27% von 18:1-Fettsäure, 59,37% von 18:2-Fettsäure und 5-30 ppm von BHT-Antioxidationsmittel, zu erhalten; wobei wahlweise der FAME eine klare gelbe Flüssigkeit mit 0,872 g/ml Dichte, 4,5 cSt (bei 40°C) Viskosität, 0,1014% gesamtem Glycerol und 0,0086% freiem Glycerol ist und der Brennwert, wie gemessen durch standardmäßigen kalorimetrischen Test, 9879 kcal/kg ist.

6. Verfahren nach Anspruch 5-6, wobei der besagte FAME in einem regulären unmodifizierten Dieselfahrzeug als B20-Gemisch unter Vollbelastungsbedingung und Einhaltung von Emissionsanforderungen verwendet wird.

7. Verfahren nach Anspruch 3, wobei die Lipidfraktion, erhalten aus *Chlorella variabilis* (ATCC-Zugangsnummer PTA 12198), raffiniert und umgeestert wird, um FAME mit einer Zusammensetzung, wie analysiert durch GC-MS, umfassend 6,9% von 16:0-Fettsäure, 3,1% von 18:0-Fettsäure; 32,6% von 18:1-Fettsäure und 57,3% von 18:2-Fettsäure und 5-30 ppm von BHT-Antioxidationsmittel, zu erhalten; wobei wahlweise der FAME eine klare senfgelbe Flüssigkeit mit einer Dichte bei 25°C und 40°C von 0,8704 bzw. 0,8591 g/cm³; Viskosität bei 40°C 4,8 cSt; gesamtem Glycerin, 0,15%; freiem Glycerin 0,02%; CFPP, Feuchtigkeitsgehalt 0,029%, -5°C; Phosphor, 5,1 ppm; Oxidationsstabilität 0,43 Jahre (25°C) und 0,12 Jahre (40°C) ist und der Brennwert, wie gemessen durch standardmäßigen kalorimetrischen Test, 9843 kcal/kg ist.

8. Verfahren nach den Ansprüchen 8-9, wobei der FAME in einem regulären unmodifizierten Dieselfahrzeug als B100-Biodiesel unter Vollbelastungsbedingung und Einhaltung von Emissionsanforderungen verwendet wird.

9. Verfahren nach den Ansprüchen 1-2, 5-7 und 11, wobei die Lipidausbeute bei Hexanextraktion für Matten von *Microspora sp.* in dem Bereich von 5,22-16,32% ist.

10. Verfahren nach den Ansprüchen 1, 3, 8-10 und 12-13, wobei die Lipidausbeute bei Hexanextraktion für die kultivierte *Chlorella variabilis* (ATCC-Zugangsnummer PTA 12198) in dem Bereich von 11,11-11,21% ist.

11. Verfahren nach den Ansprüchen 1, 3, 8-10, 12-13 und 15, wobei Wachstumsgeschwindigkeit und Lipidausbeute von *Chlorella variabilis* durch Zugabe von 3-6 kg von Natriumbicarbonat, 1-2 kg Natriumnitrat und 0,01-0,02 kg Eisen(II)-sulfat pro 1000 1 des Seewasser-Kulturmediums beeinflusst wird.

12. Verfahren nach den Ansprüchen 1, 3, 8-10, 12-13 und 15-16, wobei wahlweise rohes Glycerol aus Nebenproduktströmen des FAME-Verfahrens hinzugefügt wird, um die Biomasseproduktivität um 50-200% zu erhöhen.

13. Verfahren nach den Ansprüchen 1-18, wobei restliche Biomasse nach der Lösungsmittelextraktion von Lipid bei der Erzeugung von Biodünger, Wassertierfutter, eines Ausgangsstoffs von Carotinoiden und einer Energiequelle benutzt wird.

14. Verfahren nach den Ansprüchen 1-18, wobei Nebenproduktströme von rohem Glycerol für Algenproduktivität durch mixotrophes Wachstum und/oder für Erzeugung von biologisch abbaubarem Biopolymer benutzt werden.

## Revendications

1. Procédé pour la production d'un ester méthylique d'acide gras (FAME) digne des moteurs pour l'utilisation en tant que biodiesel, le procédé comprenant les étapes consistant:
(i) à récolter des nappes de microalgues marines naturelles choisies dans le groupe constitué par *Microspore sp.* et *Cladophora sp.* ou *Chlorelle variabilis* cultivée pour obtenir une biomasse d'algues;
(ii) à sécher la biomasse au soleil à un taux d'humidité résiduelle de 5-10%;
(iii) à prétraiter la biomasse de l'étape (ii) par jet de vapeur ou choc osmotique pour désintégrer la paroi cellulaire;
(iv) à extraire un lipide à partir de la biomasse d'algue de l'étape (iii) en utilisant de l'hexane en tant que solvant ou facultativement avec du diesel lorsque le combustible doit être utilisé sous forme de mélange pour obtenir une huile brute;
(v) à épurer l'hexane et traiter l'huile brute obtenue avec de la terre à foulon ou facultativement à traiter l'extrait de l'étape (ii) directement avec de la terre à foulon pour éliminer les phospholipides, les pigments et d'autres impuretés;
(vi) à filtrer pour éliminer les substances solides en suspension et à traiter l'extrait huileux de l'étape (v) davantage pour réduire la teneur en acides gras libres (FFA), s'il le faut pour obtenir une huile raffinée;
(vii) à réaliser une transestérification, catalysée par un alcali, de l'huile raffinée de l'étape (vi), à séparer le FAME et à le purifier davantage pour obtenir un FAME digne des moteurs.

2. Procédé selon la revendication 1, dans lequel le lipide est extrait à partir d'une nappe de microalgues marines comprenant *Microspore sp.* (numéro d'accès ATCC PTA-12197) par extraction avec de l'hexane, le lipide ayant pour composition, telle qu'analysée par CPG-SM, 0,6% d'acide gras 14:0, 9,4% d'acide gras 16:0, 0,7% d'acide gras 16:1, 3,7% d'acide gras 18:0, 33,2% d'acide gras 18:1, 50,4% d'acide gras 18:2, 0,7% d'acide gras 20:0, 1,3% d'acide gras 22:0.

3. Procédé selon la revendication 1, dans lequel le lipide est extrait à partir de microalgues marines *Chlorelle variabilis* (numéro d'accès ATCC PTA 12198), par extraction avec de l'hexane, le lipide ayant pour composition, telle qu'analysée par CPG-SM, 0,4% d'acide gras 14:0, 12,1% d'acide gras 16:0, 1,0% de 16:1, 1,0% d'acide gras 16:2, 4,2% d'acide gras 18:0, 29,4% de 18:1, 45,7% d'acide gras 18:2, 4,8% d'acide gras 18:3, 1,4% de 22:0.

4. Procédé selon la revendication 1, dans lequel le lipide est extrait à partir d'une nappe de microalgues marines comprenant *Cladophora sp.* (numéro d'accès ATCC PTA 12199), par extraction avec de l'hexane, le lipide ayant pour composition, telle qu'analysée par CPG-SM, 0,9% d'acide gras 14:0, 0,4% d'acide gras 15:0, 21,5% d'acide gras 16:0, 1% d'acide gras 16:1, 2,9% d'acide gras 18:0, 21,2% d'acide gras 18:1, 22,3% d'acide gras 18:2, 0,5% d'acide gras 20:0, 16,3% d'acide gras 20:1, 0,4% d'acide gras 22:0, 11,4% d'acide gras 22:1, 0,7% d'acide gras 24:0, 0,6% d'acide gras 24:1.

5. Procédé selon la revendication 2, dans lequel la fraction lipidique obtenue à partir de *Microspore sp.* est raffinée et transestérifiée pour obtenir un FAME ayant une composition, telle qu'analysée par CPG-SM, comprenant 9,92% d'acide gras 16:0, 2,44% d'acide gras 18:0, 28,27% d'acide gras 18:1, 59,37% d'acide gras 18:2, et 5 à 30ppm d'antioxydant BHT; facultativement dans lequel le FAME est un liquide jaune transparent ayant une masse volumique de 0,872g/ml, une viscosité de 4,5cSt (à 40°C), 0,1014% de glycérol total et 0,0086% de glycérol libre, et le pouvoir calorifique, tel que mesuré par essai calorimétrique standard, est de 9879kcal/kg.

6. Procédé selon les revendications 5-6, dans lequel ledit FAME est utilisé dans un véhicule diesel normal non modifié sous la forme d'un mélange B20 dans une condition de charge pleine et répondant aux conditions requises d'émissions.

7. Procédé selon la revendication 3, dans lequel la fraction lipidique obtenue à partir de *Chlorelle variabilis* (numéro d'accès ATCC PTA 12198) est raffinée et transestérifiée pour obtenir un FAME ayant une composition, telle qu'analysée par CPG-SM, comprenant 6,9% d'acide gras 16:0, 3,1% d'acide gras 18:0, 32,6% d'acide gras 18:1 et 57,3% d'acide gras 18:2, et 5 à 30ppm d'antioxydant BHT; facultativement dans lequel ledit FAME est un liquide jaune moutarde transparent ayant une masse volumique à 25°C et 40°C de 0,8704 et 0,8591g/cm³, respectivement; viscosité à 40°C, 4,8cSt; glycérine totale, 0,15%; glycérine libre, 0,02%; CFPP, teneur en humidité, 0,029%; -5°C; phosphore, 5,1ppm; stabilité à l'oxydation, 0,43 an (25°C) et 0,12 an (40°C), et le pouvoir calorifique, tel que mesuré par essai calorimétrique standard, est de 9843kcal/kg.

8. Procédé selon les revendications 8-9, dans lequel ledit FAME est utilisé dans un véhicule diesel normal non modifié sous la forme d'un biodiesel B100 dans une condition de charge pleine et répondant aux conditions requises d'émissions.

9. Procédé selon les revendications 1-2, 5-7 et 11, dans lequel le rendement en lipide avec extraction par hexane pour des nappes de *Microspore sp.* est dans la gamme de 5,22 à 16,32%.

10. Procédé selon les revendications 1, 3, 8-10 et 12-13, dans lequel le rendement en lipide avec extraction par hexane pour *Chlorelle variabilis* cultivée (numéro d'accès ATCC PTA 12198) est dans la gamme de 11,11-11,21%.

11. Procédé selon les revendications 1, 3, 8-10, 12-13 et 15, dans lequel la vitesse de croissance et le rendement en lipide de *Chlorelle variabilis* sont influencés par addition de 3 à 6kg de bicarbonate de sodium, de 1 à 2kg de nitrate de sodium et de 0,01 à 0,02kg de sulfate ferreux pour 1000 1 du milieu de culture d'eau de mer.

12. Procédé selon les revendications 1, 3, 8-10, 12-13 et 15-16, dans lequel du glycérol brut de courants de sous-produits du procédé FAME est facultativement ajouté pour accroître la productivité de la biomasse de 50 à 200%.

13. Procédé selon les revendications 1-18, dans lequel une biomasse résiduelle après extraction de lipide par solvant est utilisée dans la production d'un biofertilisant, d'une nourriture pour animaux aquatiques, d'une source de caroténoïdes et d'une source d'énergie.

14. Procédé selon les revendications 1-18, dans lequel des courants de co-produits de glycérol brut sont utilisés pour la productivité des algues par croissance mixotrophique et/ou pour la production de biopolymère biodégradable.
